Europäisches Patentamt

European Patent Office

Office européen des brevets

(11) Veröffentlichungsnummer: **0 279 439 B1**

# EUROPÄISCHE PATENTSCHRIFT

(45) Veröffentlichungstag der Patentschrift: **07.10.92**

(51) Int. Cl.5: **B01D 9/00**

(21) Anmeldenummer: **88102334.5**

(22) Anmeldetag: **18.02.88**

(54) Verfahren zur Reiningung ausgefrorener Kristallschichten.

(30) Priorität: **20.02.87 DE 3705388**

(43) Veröffentlichungstag der Anmeldung:
**24.08.88 Patentblatt 88/34**

(45) Bekanntmachung des Hinweises auf die
Patenterteilung:
**07.10.92 Patentblatt 92/41**

(84) Benannte Vertragsstaaten:
**BE CH DE FR GB IT LI NL**

(56) Entgegenhaltungen:
**EP-A- 0 051 551**
**US-A- 3 305 320**
**US-A- 3 323 730**
**US-A- 3 557 226**

**R.H. PERRY et al.: "Perry's chemical engineeers' handbook", 6. Auflage, 1984, Seiten
19-28 - 19-29, McGraw-Hill Book CO., New
York, US**

(73) Patentinhaber: **BASF Aktiengesellschaft
Carl-Bosch-Strasse 38
W-6700 Ludwigshafen(DE)**

(72) Erfinder: **Holzknecht, Bernhard, Dr.
Pfalzgrafenstrasse 11
W-6701 Ellerstadt(DE)**
Erfinder: **Fuchs, Hugo, Dr.
Egellstrasse 28
W-6700 Ludwigshafen(DE)**
Erfinder: **Hetzel, Eckhard
Sandweg 26
W-6712 Bobeheim-Roxheim(DE)**
Erfinder: **Wintermantel, Klaus, Dr.
Tulpenweg 1
W-6940 Weinheim(DE)**
Erfinder: **Thoma, Peter, Dr.
Peterskopfstrasse 2
W-6710 Frankenthal(DE)**

EP 0 279 439 B1

**Beschreibung**

Gegenstand der Erfindung ist ein Verfahren zur Reinigung ausgefrorener Kristallschichten.

In der DE-A-26 06 364 wird ein verbessertes Verfahren zur fraktionierten Kristallisation flüssiger Gemische beschrieben, bei dem man das flüssige Gemisch wiederholt in turbulenter Strömung durch eine indirekt gekühlte Kristallisationszone, z.B. ein Rohr leitet, mit der Maßgabe, daß die Kristallisationszone stets gefüllt ist, nach Abschieden einer Kristallschicht an der Wand der Kristallisationszone die restliche Flüssigkeit entfernt, die Oberfläche der Kristallschicht mit einem Gemisch wäscht, das der Ausgangszusammensetzung entspricht, und anschließend die Kristallschicht abschmilzt. Die Wäsche der Kristallschicht beinhaltet dabei ein Verdrängen des an der Kristalloberfläche haftenden Filmes aus restlicher Flüssigkeit durch einen Flüssigkeitsfilm aus Ausgangsmaterial. In der DE-A-1 769 123 ist auch schon ein Verfahren beschrieben, bei dem man die zu kristallisierende Schmelze als einen Rieselfilm durch indirekt gekühlte Kristallisationszonen leitet, die abgeschiedene Kristallschicht von der Restflüssigkeit trennt und anschließend abschmilzt. Bei allen drei beschriebenen Verfahren muß man zur Erzielung einer höheren Reinheit als der nach einmaligem Auskristallisieren erreichten in einer oder mehreren nachfolgenden Stufen erneut ausfrieren. Damit ist ein entsprechend erhöhter Bedarf an Apparategröße und Energie verbunden. Aus der GB-A-1 083 850 ist ein Verfahren zur fraktionierten Kristallisation bekannt, in dem man Schmelze mehrfach durch ein Rohr leitet, das Rohr kühlt und nach Entfernen der Restflüssigkeit das Kristallisat aufschmilzt. Hierbei wird der Schmelze fortlaufend Wärme zugeführt, um eine glatte Kristalloberfläche zu erhalten.

Aufgabe der Erfindung war es, die Reinigungsaufwand bei einem Verfahren zur Reinigung ausgefrorener Kristallschichten zu minimalisieren.

Diese Aufgabe wird durch die Merkmale gemäß Anspruch 1 golöst Für den Reinigungsvorgang gemäß Anspruch 1 ist keine zusätzliche Energie zum Erwärmen oder Abkühlen nötig, da die Temperatur der Kühlfläche zwischen Ausfrieren und Abschmelzen ohnehin über den Schmelzpunkt hinweg angehoben werden muß. Außerdem ist die benötigte Belegzeit des Apparates wesentlich kürzer als beim Ausfrieren, so daß der Bedarf an Apparategröße ebenfalls wesentlich kleiner ist als für eine erneute Kristallisationsstufe. Für eine geforderte Reinheit des Endproduktes sind bei jeweiliger Reinigung der ausgefrorenen Schichten weniger Kristallisationsstufen nötig als ohne Reinigung der Schichten. Dadurch sind sowohl die Kristallisatoren und die Anzahl der für jede Stufe benötigten Vorlagebehälter, als auch der Energiebedarf kleiner als ohne Reinigung.

Das Reinigungsverfahren ist anwendbar auf Schichten, die aus Schmelzen oder Lösungen ausgefroren wurden. Besondere technische Bedeutung hat die fraktionierte Kristallisation aus der Schmelze erlangt. Geeignete Stoffe sind organische Verbindungen mit einem Schmelzpunkt von -50°C bis +200°C, die sich bei den angewandten Temperaturen nicht versetzen. Geeignete Stoffe sind beispielsweise Caprolactam, Toluylendiisocyanat, Piperazin oder Naphthalin.

Die Kristallschichten, die einer Reinigung unterzogen werden, können noch auf der Kühlfläche, auf der sie aufgewachsen sind, fixiert bleiben oder sie können abgeschabt werden. Im ersten Fall läßt man die Reinigungsflüssigkeit über die Schicht hinwegströmen, im zweiten Fall ist die Kristallschicht beidseitig benetzt und ist in Teilstücken, z.B. als Schuppen, in der Reinigungsflüssigkeit suspendiert oder wird in einem Festbett von ihr durchströmt. Als Reinigungsflüssigkeit wird Schmelze oder Lösung des Produktes verwendet. Während dieses Kontaktes kommt es zu einem Reinigungseffekt durch Stofftransport des abzureinigenden Komponenten aus der Schicht in die Reinigungsflüssigkeit.

Ein wesentliches Merkmal der Erfindung ist es, daß sich die Temperatur, auf der sich die Schicht während der Reinigung befindet, in der Nähe des Schmelzpunktes bzw. der Löslichkeitstemperatur befindet. Dadurch wird der Stofftransport der Verunreinigungen begünstigt und beschleunigt. Im Falle, daß die Kristallschichten in der Reinigungsflüssigkeit suspendiert sind, wird dadurch außerdem erreicht, daß der Feststoffanteil konstant bleibt. Im Fall, daß die Kristallschichten sich noch auf der Kühlfläche befinden, wird die Temperatur der Kühlfläche, die sich beim Ausfrieren deutlich unter dem Schmelzpunkt/Löslichkeitstemperatur befindet, auf diesen Wert angehoben. Der Reinigungseffekt wird noch verstärkt, wenn man die Temperatur der Kühlfläche bis zu 5 K über die Gleichgewichtstemperatur anhebt. Dabei kann es zu einem teilweisen Anschmelzen der Kristallschichten kommen. Um im Endergebnis keinen Verlust an Kristallisat zu haben, wird die Temperatur anschließend stetig wieder etwas unter die Gleichgewichtstemperatur abgesenkt, bis der Feststoffanteil seinen Wert wieder erreicht hat.

Ein weiteres wesentliches Merkmal der Erfindung ist ein intensiver Stoffübergang an der Phasengrenze. Befinden sich die Kristallschichten noch auf den Kühlflächen, so wird die Reinigungsflüssigkeit wiederholt im Kreis durch die Kristallisationszonen geleitet. Die Kristallisationszonen können die Form von Röhren, viereckigen Kanälen oder anderen geschlossenen Profilen sowie von Platten haben. Der erforderliche intensive Stoffübergang wird in diesem Fall durch entsprechende Strömungsgeschwindigkeit bewirkt, die in der Rohrströmung, in einem Rieselfilm oder bei Überströmung einer Platte erzeugt wird. Sie liegt etwa in einem Bereich von 0,2 bis 6 m/s. Im anderen Fall von abgelösten, in der Reinigungsflüssigkeit suspendierten Kristallschichten wird der erforderliche Stoffübergang durch geeignete Maßnahmen, wie z.B. Rühren oder Umpumpen erzeugt.

Ein weiteres wesentliches Merkmal der Erfindung ist die Zeit, in der sich die Kristallschicht in Kontakt mit der Reinigungsflüssigkeit befindet. Die Geschwindigkeit des Stofftransportes nimmt von einem anfänglichen Maximalwert stetig ab. Die Reinigung kann abgebrochen werden, wenn diese Geschwindigkeit einen Grenzwert unterschreitet. Die Reinigungszeiten liegen in einem Bereich von 1 min bis 60 min. Sie hängen auch von einem weiteren Merkmal der Erfindung ab, der Schichtdicke, Schichtdicke und Reinigungszeit müssen aufeinander abgestimmt sein. Die Dicke ausgefrorener Schichten liegt in einem Bereich von 0,2 mm bis 10 mm.

Ein weiteres Merkmal der Erfindung ist die anfängliche Konzentration derjenigen Komponenten in der Reinigungsflüssigkeit, die in der Kristallschicht abgereichert werden sollen. Eine kleinere Verunreinigungskonzentration in der Reinigungsflüssigkeit führt auch zu kleineren Verunreinigungskonzentrationen in der Kristallschicht. Jedoch tritt auch eine Reinigungswirkung auf, wenn die Verunreinigungskonzentration in der Reinigungsflüssigkeit höher ist als in der Kristallschicht. Insbesondere ist nach dem Ausfrieren - bedingt durch die damit verbundene Trennwirkung - die Verunreinigungskonzentration in der Restflüssigkeit (Mutterlauge) wesentlich höher als im Kristallisat. Wenn man diese Mutterlauge anschließend als Reinigungsflüssigkeit verwendet - die Kristallschicht also in Kontakt mit der gleichen Flüssigkeit bleibt - tritt ebenfalls noch ein Reinigungseffekt auf.

Als Reinigungsflüssigkeit kann also die Mutterlauge aus dem Ausfriervorgang oder ein Teilstrom aus dem aufgeschmolzenen/aufgelösten gereinigten Kristallisat oder beides nacheinander in der Reihenfolge steigender Reinheit verwendet werden. Im Fall abgelöster und suspendierter Kristallschichten kann dies auch kontinuierlich durchgeführt werden. Die Kristallschichten werden dann im Gegenstrom zur Reinigungsflüssigkeit geführt.

Die Vorgehensweise bei dem Reinigungsverfahren im einzelnen soll an zwei Beispielen (Reinigung von Caprolactam und Isomerentrennung von Toluylendiisocyanat) erläutert werden.

Beispiel 1

Schichtkristallisation und Reinigung von Caprolactam

| Ausgangsprodukt: | |
|---|---|
| entwässertes Rohlactam, Temperatur = 76°C Schmelzpunkt ca. 69°C | |
| UV | 2579 |
| PTZ | 950 |

Die Reinheit von Caprolactam wird üblicherweise angegeben durch die UV-Zahl (UV) und die Permanganattitrationszahl (PTZ). Die Permanganattitrationszahl gibt den Verbrauch an 0,1-normaler Kaliumpermanganatlösung im ml an, berechnet auf 1 kg Caprolactam in stark saurer Lösung. Die UV-Zahl ist wie folgt definiert:

Prinzip: Im Spektralbereich von 360 bis 270 nm wird die Absorption des Caprolactams gemessen und nach Umsetzung in einer Kennzahl ausgedrückt.

Analysegerät: 1 registrierendes Einstrahlspektralphotometer (Carl Zeiss) DMR/21, 1 Erlenmeyerkolben (200 ml), 2 Quarz-Küvetten mit Deckel 10 cm lang (Schichtdicke 10 cm).

Vorschrift: 50 g Caprolactam werden in einem Erlenmeyerkolben in 50 g bidestilliertem Wasser kalt aufgelöst. Mit dieser Lösung wird eine Küvette bis zur Eichmarke gefüllt. Die zweite Küvette wird mit dem gleichen bidestillierten Wasser gefüllt und stellt die Vergleichslösung dar.

Nun werden beide Küvetten mit den Deckeln verschlossen, die geschliffenen Flächen mit Seidenpapier gereinigt und in die Küvettenhalter eingesetzt. Dann wird gemäß Geräteanleitung das Spektrum zwischen 370 nm und 260 nm aufgenommen. Die Registriergeschwindigkeit beträgt 50. Die Extinktionsmessung wird im Maßbereich 0-1 ausgeführt.

Ist die Aufnahme beendet, wird von 270 bis 360 nm alle 10 nm eine Markierung auf dem Papier angebracht.

Auswertung: Aus dem Diagramm werden Extinktionen bei 270, 280, 290, 300, 310, 320, 330, 340, 350

EP 0 279 439 B1

und 360 nm abgelesen und addiert.

Die Summe der 10 Extinktionswerte wird mit 2 multipliziert und ergibt die UV-Kennzahl. Die UV-Kennzahl wird also immer auf 100%iges Caprolactam und auf eine Schichtdicke von 10 cm bezogen.

Ablauf:

1. Füllen

Die Kristaller 1,2 werden durch den Sekundärkreislauf 10 auf eine Temperatur von 60°C gebracht. Vom letzten Kristallisationszyklus befand sich eine dünne Impfkristallschicht auf den Kühlflächen (durch einen kristallisierten Rieselfilm nach dem Entleeren der Schmelze). Das Ausgangsprodukt wird über den Anschluß 7 durch die Pumpe 5 in die Kristaller 1,2 gepumpt. Die Ventile A und B sind geschlossen, das Ventil C ist offen. Wenn in Ausgleichsbehälter 4 die Standmessung anspricht, wird der Füllvorgang beendet. Während des Füllvorgangs (Dauer ca. 1 min) wird die Sekundärkreistemperatur von 60°C auf 64°C angehoben. Eingefüllte Menge: 12,34 kg Ausgangsprodukt.

2. Kristallisieren

Nach Beendigung des Füllens wird die Schmelze durch den Kristaller 1, die Verbindung 3, den Kristaller 2 und die Pumpe 5 im Kreis gepumpt. Dabei wird die Sekundärkreistemperatur in 3 min von 64°C auf 66°C angehoben, dann in 75 min von 66°C auf 51°C abgesenkt. Der umgewältze Volumenstrom beträgt anfangs 1,47 m³/h. Durch Auskristallisieren der Kristallschicht auf der Rohrinnenseite und infolgedessen teilweiser Versperrung des Strömungsquerschnitts sinkt der umgewälzte Volumenstrom. Der Kristallisiervorgang wird beendet, wenn der Volumenstrom einen Grenzwert von 0,30 m³/h erreicht hat. Die Zeit für den Kristallisationsvorgang beträgt 78 min.

3. Reinigung mit Mutterlauge

Die Sekundärkreistemperatur wird auf 72°C angehoben. Der Volumenstrom nimmt leicht zu (0,45 m³/h). Nach kurzer Verweilzeit wird die Temperatur wieder abgesenkt auf ca 51°C, bis der Volumenstrom wieder den Endwert nach dem Kristallisieren von 0,30 m³/h erreicht hat. Die Dauer dieses Schrittes beträgt 10 min.

Die Mutterlauge wird jetzt über den Anschluß 7 abgelassen.
Menge: 7,31 kg

| Mutterlauge: | |
|---|---|
| UV | 3958 |
| PTZ | 1500 |

4. Reinigung mit Reinlactam

| Reinlactam vor Reinigung: | |
|---|---|
| UV | 870 |
| PTZ | 385 |

Die Sekundärkreistemperatur wird auf 71°C angehoben. Über den Anschluß 7 und die Pumpe 5 wird 6,99 kg Reinlactam zur Reinigung eingefüllt. Es wird anschließend 20 min umgewälzt. Dabei steigt der Volumenstrom auf 0,45 m³/h an. Gegen Ende des Reinigungschrittes wird die Sekundärkreistemperatur wieder auf ca. 48°C abgesenkt, so daß der Volumenstrom wieder 0,30 m³/h erreicht. Es wird jetzt das Reinlactam über den Anschluß 7 abgelassen: Menge 7,02 kg.

4

| Reinlactam nach Reinigung: | |
| --- | --- |
| UV | 925 |
| PTZ | 385 |

### 5. Schmelzen und Entleeren

Das Kristallisat wird nun aufgeschmolzen durch Erhöhen der Sekundärkreistemperatur über den Schmelzpunkt. Dies geschieht, damit eine Probenahme ohme Vermischung möglich ist. Sonst wird aufgeschmolzenes Kristallisat eingefüllt, umgepumpt und die Schmelzwärme über den Wärmetauscher 9 zugeführt. Nach Aufschmelzen wird das Kristallisat über den Anschluß 7 abgelassen. Die Kristallisatmenge betrug 5 kg.

| Kristallisat: | |
| --- | --- |
| UV | 499 |
| PTZ | 230 |

Der Sekundärkreislauf wird unmittelbar nach Ablassen des Kristallisates möglichst schnell ca. 20 K unter dem Schmelzpunkt auf 50°C gefahren, um den an den Kühlflächen haftenden Rieselfilm als Impfkristallschicht für den nächsten Zyklus durchzukristallisieren.

Vergleichsweise hierzu betragen die Werte für ein

| Kristallisat ohne Reinigung: | |
| --- | --- |
| UV | 1033 |
| PTZ | 375 |

### Beispiel 2

Schichtkristallisation und Reinigung von Toluylendiisocyanat (TDI)-Isomeren zur Isomerentrennung

| Ausgangsprodukt: | |
| --- | --- |
| 80,24 % | 2,4-TDI |
| 19,76 % | 2,6-TDI |
| Temperatur: 21,4°C (Schmelzpunkt) | |

Ablauf:

### 1. Füllen

Die Kristaller 1,2 werden durch den Sekundärkreislauf 10 auf eine Temperatur von 6°C gebracht. Von letzten Kristallisationszyklus befand sich eine dünne Impfkristallschicht auf den Kühlflächen (durch kristallisierten Rieselfilm nach dem Entleeren der Schmelze). Das Ausgangsprodukt wird über den Anschluß 7 durch die Pumpe 5 in die Kristaller 1,2 gepumpt. Die Ventile A und B sind geschlossen, das Ventil C ist offen. Wenn im Ausgleichsbehälter 4 die Standmessung anspricht, wird der Füllvorgang beendet. Während des Füllvorgangs (Dauer ca. 1 min) wird die Sekundärkreistemperatur von 6 auf 7°C angehoben. Eingefüllte Menge: 16 kg.

### 2. Kristallisieren

Nach Beendigung des Füllens wird die Schmelze durch den Kristaller 1, die Verbindung 3, den Kristaller 2 und die Pumpe 5 in Kreis gepumpt. Dabei wird die Sekundärkreistemperatur in 3 min von 7°C auf 10°C, in 6 min von 10°C auf 13°C angehoben, dann 40 min auf 13°C gehalten, in 4 h auf 9°C und dann in weiteren 71 min auf 3°C abgesenkt. Der umgewälzte Volumenstrom beträgt anfangs 1,67 m$^3$/h. Durch Auskristallisieren der Kristallschicht auf der Rohrinnenseite und infolgedessen teilweiser Versperrung des Strömungsquerschnitts sinkt der umgewälzte Volumenstrom.

Der Kristallisiervorgang wird beendet, wenn der Volumenstrom einen Grenzwert von 0,41 m$^3$/h erreicht hat. Zeit für das Kristallisieren = 360 min.

Die Mutterlauge wird jetzt über den Anschluß 7 abgelassen. Menge: 11 kg

| Mutterlauge: | |
|---|---|
| 74,79 % | 2,4-TDI |
| 25,21 % | 2,6-TDI |

3. Reinigung mit 2,4-TDI

| Reinigungsflüssigkeit vor Reinigung: | |
|---|---|
| 99,32 % | 2,4-TDI |
| 0,68 % | 2,6-TDI |

Die Sekundärkreistemperatur wird auf 19°C angehoben. Über den Anschluß 7 und die Pumpe 5 wird 11 kg Reinigungsflüssigkeit eingefüllt. Es wird anschließend 45 min umgewälzt. Dabei beträgt der Volumenstrom 0,78 m$^3$/h. Gegen Ende des Reinigungsschrittes wird die Sekundärkreistemperatur wieder auf ca. 15°C abgesenkt, so daß der Volumenstrom 0,74 m$^3$/h erreicht. Es wird jetzt das 2,4-TDI über den Anschluß 7 abgelassen: Menge 11 kg.

| Reinigungsflüssigkeit nach Reinigung: | |
|---|---|
| 96,99 % | 2,4-TDI |
| 3,01 % | 2,6-TDI |

4. Schmelzen und Entleeren

Das Kristallisat wird nun aufgeschmolzen durch Erhöhung der Sekundärkreistemperatur über den Schmelzpunkt; dies geschieht, damit eine Probenahme ohne Vermischung möglich ist. Sonst wird aufgeschmolzenes Kristallisat eingefüllt, umgepumpt und die Schmelzwärme über den Wärmetauscher 9 zugeführt. Nach Aufschmelzen wird das Kristallisat über den Anschluß 7 abgelassen: Menge 5 kg.

| Kristallisat: | |
|---|---|
| 98,33 % | 2,4-TDI |
| 1,67 % | 2,6-TDI |

Die Sekundärkreislauf wird unmittelbar nach Ablassen des Kristallisates möglichst schnell ca. 20 K unter den Schmelzpunkt auf 0°C gefahren, um den an den Kühlflächen haftenden Rieselfilm als Impfkristallschicht für den nächsten Zyklus durchzukristallieren.

Aus anderen Versuchen ohne Reinigung mit 2,4-TDI ergeben sich für das

| Kristallisat: | |
|---|---|
| 92,55 % | 2,4-TDI |
| 7,45 % | 2,6-TDI |

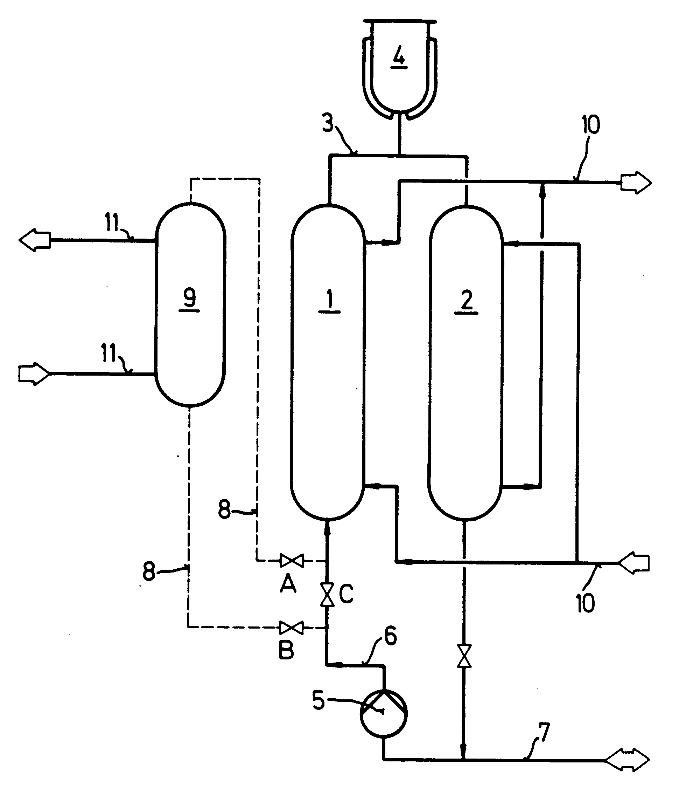

**Patentansprüche**

1. Verfahren zur Reinigung ausgefrorener Kristallschichten, bei dem diese Kristallschichten, nachdem sie aus einer Schmelze oder Lösung auf einer Kühlfläche ausgefroren wurden, mit einer Reinigungsflüssigkeit in Kontakt gebracht und damit über ihre gesamte Dicke gereinigt werden, wobei der Stoffübergang an der Phasengrenze durch erzwungene Konvektion intensiviert wird, die Temperatur der Kristallschichten und der Reinigungsflüssigkeit um den Schmelzpunkt bzw. die Löslichkeitstemperatur gehalten wird, diese Reinigung in einer Zeit von 1 min bis 60 min an Schichten mit einer Dicke von 0,2 mm bis 10 mm durchgeführt wird, und dann die Kristallschichten von der Reinigungsflüssigkeit abgetrennt und anschließend aufgeschmalzen werden.

2. Verfahren nach Anspruch 1 und 2, dadurch gekennzeichnet, daß die Kühlfläche eine ebene oder zylinderische Fläche (Kühlwalze, Innen-oder Außenseite eines Rohres) ist.

3. Verfahren nach Anspruch 1 bis 3, dadurch gekennzeichnet, daß die Kristallschichten bei der Reinigung auf der Kühlfläche haften und dabei von der Reinigungsflüssigkeit einseitig benetzt werden, oder daß sie von der Kühlfläche abgelöst werden und von der Reinigungsflüssigkeit beidseitig benetzt werden.

4. Verfahren nach Anspruch 1 bis 4, dadurch gekennzeichnet, daß die Konzentration an abzureichernden Komponenten in der Reinigungsflüssigkeit kleiner oder größer als in der Kristallschicht sein kann.

5. Verfahren nach Anspruch 1 bis 5, dadurch gekennzeichnet, daß als Reinigungsflüssigkeit die Restflüssigkeit (Mutterlauge) aus der vorangegangenen Kristallisation oder eine neu mit der Kristallschicht in Kontakt gebrachte Schmelze/Lösung verwendet wird, wobei letztere das aufgeschmolzene/aufgelöste gereinigte Kristallisat sein kann.

6. Verfahren nach Anspruch 1 bis 6, dadurch gekennzeichnet, daß eine Reinigungsflüssigkeit allein oder mehrere nacheinander in der Reihenfolge steigender Reinheit verwendet werden, oder daß Kristallschichten und Reinigungsflüssigkeit kontinuierlich im Gegenstrom zueinander geführt werden.

7. Verfahren nach Anspruch 1 bis 7, dadurch gekennzeichnet, daß während des Reinigungsvorganges die Temperatur der Kristallschicht zuerst bis zu 5 K über die Gleichgewichtstemperatur (Schmelzpunkt oder Löslichkeit) stetig angehoben wird und am Ende bis etwas unter die Gleichgewichtstemperatur wieder stetig abgesenkt wird.

**Claims**

1. A process for the purification of frozen crystal layers, in which the said layers, after they have been frozen out from a melt or solution on a cooling surface, are brought into contact with a purifying liquid and thus purified over their entire thickness, the mass transfer at the phase boundary being intensified by forced convection, the temperature of the crystal layers and of the purifying liquid being maintained at the melting point or solubility temperature, and this purification being carried out in the course of from 1 to 60 minutes on layers having a thickness of from 0.2 to 10mm, after which the crystal layers are separated from the purifying liquid and then melted.

2. A process as claimed in claim 1, wherein the cooling surface is a flat or cylindrical surface (cooling roll, inside or outside of a pipe).

3. A process as claimed in claim 1 or 2, wherein the crystal layers adhere to the cooling surface during purification and are wet on one side by the purifying liquid, or they are detached from the cooling surface and are wet on both sides by the purifying liquid.

4. A process as claimed in any of claims 1 to 3, wherein the concentration, in the purifying liquid, of components to be removed may be less than or greater than that in the crystal layer.

5. A process as claimed in any of claims 1 to 4, wherein the purifying liquid used is the residual liquid (mother liquor) from the preceding crystallization, or a fresh melt/solution brought into contact with the crystal layer, and the latter may be the molten/dissolved purifying crystals.

6. A process as claimed in any of claims 1 to 5, wherein a purifying liquid is used alone or a plurality of purifying liquids are used in succession in order of increasing purity, or crystal layers and purifying liquid are fed continuously in countercurrent with respect to one another.

7. A process as claimed in any of claims 1 to 6, wherein the temperature of the crystal layer is initially steadily increased to up to 5 K above the equilibrium temperature (melting point or solubility temperature) during the purification process, and is steadily reduced again at the end to slightly below the equilibrium temperature.

**Revendications**

1. Procédé pour purifier des couches de cristaux congelées dans lequel on met ces couches de cristaux, formées à partir d'une masse fondue ou d'une solution sur une surface refroidissante, en contact avec un liquide de purification et on les purifie ainsi sur toute leur épaisseur, le transfert de matières à la limite des phases étant intensifié par convection forcée, on maintient la température des couches de cristaux et du liquide de purification au point de fusion ou à la température de solubilité, on procède à cette purification en une durée de 1 min à 60 min sur des couches de 0,2 mm à 10 mm d'épaisseur puis on sépare les couches de cristaux du liquide de purification et on les fond.

2. Procédé selon la revendication 1, caractérisé en ce que la surface de refroidissement est une surface plane ou cylindrique (cylindre refroidisseur, face intérieure ou extérieure d'un tube).

3. Procédé selon la revendication 1 ou 2, caractérisé en ce que les couches de cristaux, lors de la purification, adhèrent à la surface refroidissante et sont donc mouillées sur une face par le liquide de purification, ou bien elles sont détachées de la surface refroidissante et sont mouillées sur les deux faces par le liquide de purification.

4. Procédé selon les revendications 1 à 3, caractérisé en ce que la concentration en les composants à éliminer dans le liquide de purification peut être inférieure ou supérieure à la concentration dans la couche de cristaux.

5. Procédé selon la revendication 1 à 4, caractérisé en ce que l'on utilise en tant que liquide de purification le liquide résiduel (liqueurs-mères) de la cristallisation précédente ou une masse fondue/solution, à nouveau mise en contact avec la couche de cristaux, cette masse fondue/solution pouvant consister en les cristaux purifiés fondus/dissous.

6. Procédé selon les revendications 1 à 5, caractérisé en ce que l'on utilise un liquide de purification seul ou plusieurs liquides de purification successivement à pureté croissante, ou bien on envoie les couches de cristaux et le liquide de purification à contre-courant l'un de l'autre en continu.

7. Procédé selon les revendications 1 à 6, caractérisé en ce que, au cours de l'opération de purification, la température de la couche de cristaux est d'abord portée en continu jusqu'à 5 K au-dessus de la température d'équilibre (point de fusion ou solubilité) puis, à la fin, à nouveau abaissée en continu jusqu'à un niveau légèrement inférieur à la température d'équilibre.